(19) Europäisches Patentamt

European Patent Office

Office européen des brevets

(11) **EP 1 294 445 B1**

(12) **EUROPÄISCHE PATENTSCHRIFT**

(45) Veröffentlichungstag und Bekanntmachung des Hinweises auf die Patenterteilung:
**22.09.2004 Patentblatt 2004/39**

(21) Anmeldenummer: **01984311.9**

(22) Anmeldetag: **02.07.2001**

(51) Int Cl.$^7$: **A61N 5/10**

(86) Internationale Anmeldenummer:
**PCT/EP2001/007553**

(87) Internationale Veröffentlichungsnummer:
**WO 2002/007817 (31.01.2002 Gazette 2002/05)**

(54) **VORRICHTUNG ZUR BESTRAHLUNG EINES TUMORGEWEBES**

DEVICE FOR IRRADIATING A TUMOR TISSUE

DISPOSITIF POUR IRRADIER UN TISSU TUMORAL

(84) Benannte Vertragsstaaten:
**AT BE CH CY DE DK ES FI FR GB GR IE IT LI LU MC NL PT SE TR**

(30) Priorität: **30.06.2000 DE 10031074**

(43) Veröffentlichungstag der Anmeldung:
**26.03.2003 Patentblatt 2003/13**

(73) Patentinhaber: **Gesellschaft für Schwerionenforschung mbH 64291 Darmstadt (DE)**

(72) Erfinder:
- **KRAFT, Gerhard**
  **64291 Darmstadt (DE)**
- **WEBER, Ulrich**
  **64291 Darmstadt (DE)**

(74) Vertreter: **Boeters, Hans Dietrich, Dr. et al Patentanwälte Boeters & Bauer, Bereiteranger 15 81541 München (DE)**

(56) Entgegenhaltungen:
WO-A-99/27839          US-A- 5 039 867
US-A- 5 668 371        US-A- 5 673 300

- LUDEWIGT B A: "ACCELERATED HELIUM-ION BEAMS FOR RADIOTHERAPY AND STEREOTACTIC RADIOSURGERY" MEDICAL PHYSICS, AMERICAN INSTITUTE OF PHYSICS. NEW YORK, US, Bd. 18, Nr. 1, 1991, Seiten 36-42, XP000220434 ISSN: 0094-2405

EP 1 294 445 B1

**Beschreibung**

Die Erfindung betrifft eine Vorrichtung zur Bestrahlung eines Tumorgewebes eines Patienten mittels Ionenstrahl gemäß dem unabhängigen Anspruch.

Die jüngst entwickelten Ionenstrahl-Abtastvorrichtungen und - verfahren, wie sie beispielsweise der europäischen Patentanmeldung EP-0986 070-A zugrundeliegen, erlauben eine erhöhte Präzision der Bestrahlung von tiefliegenden Tumoren.

Mit diesen Vorrichtungen und Verfahren wird das Zielvolumen, wie ein Tumor eines Patienten, in Schichten gleicher Reichweite zerlegt, die dann mit einem Ionenstrahl flächig rasterförmig abgetastet werden. Dieser Ionenstrahl wird in bezug auf ein ortsfestes Koordinatensystem in einen Behandlungsraum geliefert, wobei der Raumwinkel einer Ionenstrahlachse im Bestrahlungsraum festliegt oder mittels einer Gantry aus unterschiedlichen Raumwinkeln abgegeben werden kann.

Um den Tumor eines Patienten in diesem ortsfesten Koordinatensystem des Bestrahlungsraumes zu positionieren, ist es erforderlich, den Patienten zu Beginn in die richtige Sollposition in bezug auf dieses Koordinatensystem zu bringen, so daß das tatsächlich bestrahlte bzw. abgetastete Volumen des Ionenstrahls mit dem geplanten Zielvolumen des Tumors im Patienten übereinstimmt. Darüber hinaus ist es bei diesen bekannten Systemen erforderlich, die Sollposition des Patienten während der Bestrahlung einzuhalten. Zur Einhaltung der Sollposition werden aufwendige Einrichtungen wie individuell angefertigte thermoplastische Maskensysteme zur Fixierung des Patienten verwendet, um den Patienten vor der Bestrahlung millimetergenau zu justieren und während der Bestrahlung durch die Maske ruhigzustellen. Mit den bekannten Vorrichtungen und Verfahren können demnach nur räumlich fixierte Zielvolumina bestrahlt werden, wie z.B. Tumore im Kopf- und Halsbereich und Tumore nahe der Wirbelsäule, bei denen entweder der Kopf allein durch eine geeignete Maske fixiert wird oder eine Ganzkörpermaske die Wirbelsäule ruhigstellt.

Eine Bestrahlung von bewegten Zielvolumina, z.B. im Thoraxbereich, ist mit derartigen Verfahren bisher nicht möglich. Beispielsweise wird durch eine Atembewegung das Zielvolumen in einem Thoraxbereich um einige Zentimeter verschoben, und damit wird die angestrebte Millimeterpräzision unmöglich gemacht. So ist es unmöglich, eine Fixierung mit einer Genauigkeit von Millimetern zu erreichen, wenn gleichzeitig interne Bewegungen das Zielvolumen im Zentimeterbereich verschieben. Außerdem bewirkt eine Bewegung des Zielvolumens bei gleichzeitiger Strahlabtastung starke Dosisinhomogenitäten.

Während die relativ schnelle, flächige und rasterförmige Abtastung bei konstanter Energie der Ionen im Ionenstrahl den seitlichen Bewegungen des Zielvolumens im Zentimeterbereich zeitlich folgen könnte, ist die Energievariation durch den Beschleuniger nicht ausreichend schnell, um den Organbewegungen, beispielsweise durch Atmung oder Herzschlag im Thoraxbereich eines Tumorpatienten, in der Tiefe folgen zu können.

Aufgabe der Erfindung ist es, eine Vorrichtung zur Bestrahlung eines Tumorgewebes eines Patienten mittels Ionenstrahl anzugeben, bei dem der Ionenstrahl an räumliche und zeitliche Änderung, insbesondere räumliche und zeitliche periodische Änderungen des Zielvolumens sowohl senkrecht zur Strahlrichtung als auch in der Tiefe anpaßbar ist.

Gelöst wird diese Aufgabe durch den Gegenstand des unabhängigen Anspruchs. Merkmale bevorzugter Ausführungsformen werden in den abhängigen Ansprüchen definiert.

Erfindungsgemäß weist die Vorrichtung zur Bestrahlung eines Tumorgewebes eines Patienten mittels Ionenstrahl eine Ablenkeinrichtung des Ionenstrahls zum scheibenweisen Flächenabtasten des Tumorgewebes und einen Beschleuniger mit einer Ionenstrahl-Energiesteuereinrichtung zur stufenweisen Tiefenabtastung des Ionenstrahls auf. Darüber hinaus weist die Vorrichtung eine Ionen-Abbremseinrichtung auf, die als eine Tiefenabtast-Anpassungsvorrichtung zur Anpassung der Reichweite des Ionenstrahls eingesetzt ist und eine schnellere Tiefenanpassung als die Energiesteuereinrichtung des Beschleunigers aufweist. Ferner besitzt die Vorrichtung eine Bewegungserfassungseinrichtung zur Erfassung einer zeitlichen und örtlichen Änderung der Lage des Tumorgewebes in einem Behandlungsraum und eine Steuereinrichtung, welche die Ablenkeinrichtung und die Tiefenabtast-Anpassungsvorrichtung zur Nachführung der Ionenstrahlrichtung bzw. Ionenstrahlreichweite beim Abtasten des Tumorgewebes unter zeitlicher und örtlicher Lageänderung des Tumorgewebes im Behandlungsraum steuert.

Die erfindungsgemäße Vorrichtung hat den Vorteil, daß bewegte Zielvolumina eines sich bewegenden Patienten mit derselben Präzision wie unbewegte Zielvolumina in einem fixierten Patienten bestrahlt werden können. Dazu erfaßt die Bewegungserfassungseinrichtung die Bewegungen des Patienten während der Bestrahlung und die Bestrahlungspunkte werden mit Hilfe der Steuereinrichtung entsprechend korrigiert. Im Prinzip erübrigt sich bei dieser Vorrichtung auch eine anfängliche millimetergenaue Justierung des Patienten in den raumfesten Koordinaten, da mit Hilfe der Bewegungserfassungseinrichtung auch die tatsächliche Anfangslage eines Patienten an das Bestrahlungsprogramm angepaßt bzw. das Bestrahlungsprogramm entsprechend korrigiert werden kann.

In einer bevorzugten Ausführungsform der Erfindung weist die Vorrichtung zwei Elektromagnete auf, womit die Ablenkvorrichtung ein flächiges Abtasten ermöglicht. Diese Elektromagnete lenken den Ionenstrahl orthogonal zur Ionenstrahlachse in einer X- und einer Y-Richtung ab, welche ihrerseits senkrecht zueinander liegen, um scheibenweise ein relativ zur Tiefenabtastung mittels Ionenstrahl-Energiesteuereinrichtung schnelles flächiges Abtasten des Tumorgewebes zu gewährleisten. Dazu werden die Elektromagnete durch reaktionsschnelle Netzteile und Meßgeräte gesteuert. Diese Geräte können somit auch genutzt werden, um eine Korrektur und Anpassung beim Abtasten eines Tumorgewebes unter zeitlicher und örtlicher Lageveränderung des Tumorgewebes im Behandlungsraum orthogonal zur Ionenstrahlachse zu erreichen.

In einer bevorzugten Ausführungsform der Erfindung weist die Vorrichtung mindestens einen Beschleuniger auf, mit dem die Energie des Ionenstrahls einstellbar ist, so daß das Tumorgewebe scheibenweise in der Tiefe gestaffelt bestrahlbar ist.

Damit ist der Vorteil verbunden, daß nacheinander das gesamte Tumorgewebe scheibenweise abtastbar wird, wobei von Scheibe zu Scheibe die Reichweite des Ionenstrahls durch Änderung der Energie des Ionenstrahls einstellbar ist. Der Beschleuniger besteht zu diesem Zweck im wesentlichen aus einem Synchrotron oder einem Synchrozyklotron, in dem Ionen gleicher Masse und gleicher Energie stufenweise auf höhere Energien beschleunigt werden können. Die Energieanpassung des Ionenstrahls an vorgegebene Reichweiten innerhalb des Bestrahlungsraums bzw. innerhalb des Tumorvolumens ist aufgrund der Komplexität der Steuerungsfunktionen für den Beschleuniger nicht derart kurzfristig und mit der geforderten Präzision anpaßbar, daß den Bewegungen des Tumorgewebes bzw. des Patienten automatisch gefolgt werden kann.

In einer bevorzugten Ausführungsform der Erfindung weist deshalb die Tiefenabtast-Anpassungsvorrichtung zwei im Querschnitt keilförmige Ionen-Abbremsplatten auf, die das gesamte Bestrahlungsfeld des Ionenstrahls abdekken und eine schnelle Tiefenabtastanpassung bei bewegtem Tumorgewebe ermöglichen.

Dazu sind in einer bevorzugten Ausführungsform der Erfindung die Ionen-Abbremsplatten auf elektromagnetisch betätigbaren Schlitten angeordnet. Mit Hilfe dieser elektromagnetisch betätigbaren Schlitten kann die Position der keilförmigen Ionen-Abbremsplatten innerhalb von Millisekunden verändert werden und damit die in einem Überlappungsbereich der keilförmigen Abbremsplatten auftretende Länge des Abbremsweges der Ionen durch die Ionen-Abbremsplatten variiert werden. Dazu überlappen sich die Ionen-Abbremsplatten im gesamten Bestrahlungsfeld des Ionenstrahls und können somit ortsunabhängig die Ionen in ihrer Reichweite an örtliche und zeitliche Änderungen eines bewegten Zielvolumens anpassen.

In einer bevorzugten Ausführungsform der Erfindung sind die Ionen-Abbremsplatten auf Linearmotoren montiert. Derartige Linearmotoren haben den Vorteil, daß eine kontinuierliche Feinregulierung der Ionenabbremsung zur Anpassung der Tiefenabtastung des Zielvolumens möglich ist. Darüber hinaus ist die Verstellung der Position der keilförmigen Ionen-Abbremsplatten mit Hilfe von Linearmotoren nicht nur örtlich äußerst präzise, sondern auch äußerst reaktionsschnell an zeitlicher Verschiebung des Zielvolumens in der Tiefe anpaßbar.

In einer weiteren Ausführungsform wird statt der Keile ein mit Wasser gefüllter Zylinder, dessen Dicke variiert, verwendet. Die Deckel des Zylinders bestehen aus transparenten Platten, z.B. aus zwei Plexi- oder Quarzglas-Scheiben, von denen die obere Scheibe durch 2 oder 4 leistungsstarke Linearmotoren bewegt wird. Die seitliche Abdeckung des Zylinders wird durch einen Faltenbalg aus Stahl oder Gummi realisiert. Die Variation der Dicke der Wasserschicht wird durch ein Hydrauliksystem unterstützt, das beim Auseinanderziehen des Zylinders Wasser in den Zylinder hineinpumpt und beim Zusammendrücken Wasser heraussaugt, damit der Antrieb entlastet und die Bildung von Vakuolen verhindert wird.

Diese Ausführungsform hat den Vorteil, daß eine kleinere Minimaldicke als bei den Keilen möglich ist. Bei den Keilen berechnet sich die Minimaldicke aus Keil-Steigung x Feldgröße (typischerweise 5 cm). Bei der Zylinderkonstruktion ist die Minimaldicke durch die Dicke der beiden Deckel gegeben (typischerweise 1 cm). Diese geringe Minimaldicke verringert die Aufstreuung des Strahls und verbessert somit die Strahlqualität. Ferner ist die Zylinderkonstruktion in Querrichtung kompakter als die Keilkonstruktion.

In einer weiteren bevorzugten Ausführungsform der Erfindung weist die Bewegungserfassungseinrichtung mindestens zwei Meßaufnehmer auf, die aus zwei Raumwinkeln in bezug auf eine Ionenstrahlachse die zeitliche und örtliche Lage von Markierungen auf einem, ein Tumorgewebe enthaltenen Körperbereich eines Patienten erfassen. Derartige Markierungen können mit hautverträglichen Leuchtfarben in Form von Punkten, Strichen oder anderen geometrischen Formen oder als Leuchtelemente angebracht werden, um deutlich von den Meßaufnehmern wahrgenommen und vermessen zu werden.

In einer weiteren bevorzugten Ausführungsform der Erfindung sind die Meßaufnehmer Präzisionsvideokameras, die mit einer Bildauswerteinheit zusammenwirken. Damit wird vorteilhaft erreicht, daß die Bewegungen eines Körperbereichs in der Nähe eines Tumorgewebes exakt vermessen werden können und mit den zeitlichen und örtlichen Lageverschiebungen des Tumorgewebes korreliert werden können.

Alternativ zu dem Bewegungserfassungssystem, welches Markierungen auf der Körperoberfläche und ein Präzisionsvideosystem verwendet, weist eine weitere Ausführungsform der Erfindung ein Röntgensystem auf, das die Bewegungen des Tumorgewebes direkt im Körper erfaßt. Bei diesem Bewegungserfassungssystem werden zwei Röntgenröhren mit Strahlrichtung orthogonal zum Ionenstrahl angebracht. Die beiden Röntgenröhren sind ihrerseits auch senkrecht zueinander orientiert. Dazu werden zwei jeweils gegenüberliegende, auf der anderen Seite des Patienten empfindliche Röntgenbildverstärker angebracht. Die Röntgenröhren emittieren kurze Röntgenblitze mit niedriger Leistung, um die Dosisbelastung gering zu halten, mit einer Frequenz von beispielsweise 20 Hz. Die zugehörigen Röntgenbilder werden von den Bildverstärkern aufgezeichnet und digitalisiert. Dadurch wird eine Bildfolge für zwei Richtungen erhalten, aus denen mit einem geeignetem Verfahren und entsprechender Software die Verschiebung der Zielpunkte $P_i$ in nahezu Echtzeit bei etwa 50 ms Verzögerung ermittelt wird.

Diese Ausführungsform hat den Vorteil, daß von den Röntgenaufnahmen viel mehr Informationen über die Bewegungen im Körperinneren erhalten werden als von äußeren Markierungen auf der Körperoberfläche. Dies erlaubt eine präzisere Bestimmung der zeitlichen und örtlichen Organverschiebungen.

Im Prinzip setzt sich die Bestrahlung des Tumorvolumens aus Bildpunkten zusammen, die flächig in einer Scheibenform rasterförmig aneinandergesetzt werden, wobei der Ionenstrahl von Abtastpunkt zu Abtastpunkt orthogonal zu seiner Strahlachse in einer X- und einer Y-Richtung abgelenkt wird. Wenn auch die Energie der Ionen in einem Ionenstrahl durch den entsprechenden Beschleuniger konstant gehalten werden kann, so ist doch die Anzahl der Ionen pro Volumenpunkt über die Zeit nicht konstant. Um dennoch eine gleich große Ionenstrahldosis in jedem Volumenpunkt des Tumorgewebes einzustrahlen, ist in einer bevorzugten Ausführungsform der Erfindung eine Ionisationskammer mit schneller Auslese zur Überwachung der Intensität des Ionenstrahlstromes als Transmissionszähler im Strahlengang des Ionenstrahls angeordnet. Ein derartiger Transmissionszähler bestimmt die Verweilzeit des Ionenstrahls auf einem zu bestrahlenden Volumenpunkt des Tumorvolumens, und eine damit verbundene Steuereinheit lenkt den Ionenstrahl zum nächsten Volumenpunkt ab, sobald eine vorgegebene Strahlendosis erreicht ist. Somit kann in vorteilhafter Weise eine Volumenscheibe eines Tumorvolumens in flächiger Weise rasterförmig abgetastet werden.

Vorzugsweise ist die Ionisationskammer zwischen der Ablenkeinrichtung und der Tiefenabtast-Anpassungsvorrichtung angeordnet, zumal die Tiefenabtast-Anpassungsvorrichtung mit ihren keilförmigen Ionen-Abbremsplatten oder der Wasserschicht zwischen transparenten Platten lediglich die Ionen in ihrer Reichweite steuert, nicht aber die Ionendosis beeinflußt.

Ein Verfahren zur Bestrahlung eines Tumorgewebes eines Patienten mittels Ionenstrahl zur Anwendung der erfindungsgemäßen Vorrichtung weist folgende Verfahrensschritte auf:

- Lagerung des Patienten auf einer der Patientenkontur angepaßten Vorrichtung zum Plazieren des Patienten in einem Bestrahlungsraum,
- Anbringen von Markierungen auf einem Körperbereich des Patienten nahe dem Tumorgewebe,
- Erfassen der zeitlichen und örtlichen Änderung der Markierungen über eine Bewegungserfassungseinrichtung oder Erfassen von Röntgenbildern des Tumorgewebes aus zwei senkrecht aufeinanderstehenden Richtungen von Röntgenstrahlen orthogonal zum Ionenstrahl,
- Nachführen des Ionenstrahls beim Abtasten des Tumorgewebes mit einer Ionenstrahl-Ablenkeinrichtung und einer Ionenstrahl-Energiesteuereinrichtung mittels einer zusätzlichen Tiefenabtast-Anpassungsvorrichtung, welche die Reichweite des Ionenstrahls an die durch die Bewegungserfassungseinrichtung erfaßten zeitlichen und örtlichen Änderungen der Markierungen im Zusammenwirken mit der Ionenstrahl-Ablenkeinrichtung anpaßt.

Mit diesem Verfahren wird es in vorteilhafter Weise möglich, die gleiche Präzision wie beim fixierten Patienten auch beim bewegten Patienten im Millimeterbereich bei der Bestrahlung von bewegten Tumorvolumina zu erreichen, selbst wenn das Tumorgewebe bis zu mehreren Zentimetern periodisch, beispielsweise durch Herzschlag oder Beatmungsluft, bewegt wird. Dabei folgt die Ionenstrahlbestrahlung ständig der zeitlichen und örtlichen Lageverschiebung des Tumorgewebes und es muß nicht mit der Bestrahlung abgewartet werden, bis eine sich wiederholende örtliche Lage erreicht ist. Auch langsame Bewegungen des Patienten, die nichtperiodisch ablaufen, sind zulässig und können mit Hilfe der Tiefenanpassungsvorrichtung und der Ablenkeinrichtung zeitlich und örtlich in ihrer Ionenbestrahlung angepaßt werden. Lediglich bei plötzlichen Lageverschiebungen wie bei Hustenanfällen muß der Bestrahlungsvorgang abgebrochen werden.

Gegenüber Verfahren, die nur ein Bestrahlen bei Erreichen identischer Lagen des Tumorgewebes zulassen, hat das Verfahren den Vorteil, daß die Bestrahlungszeit eines Patienten wesentlich verkürzt werden kann, da der Bestrahlungsablauf beispielsweise nicht von der Periodizität des Herzschlags oder der Atmung eines Patienten abhängig ist.

Weitere Vorteile und Merkmale der vorliegenden Erfindung werden nun anhand von Ausführungsformen mit Bezug auf die beiliegenden Zeichnungen näher erläutert.

Fig. 1 zeigt eine Prinzipskizze einer Ausführungsform der Erfindung unter Bestrahlung eines Tumorgewebes im Thoraxbereich eines Patienten.

Fig. 2 zeigt eine Prinzipskizze einer Ausführungsform einer Bewegungserfassungseinrichtung.

Fig. 3 zeigt einen Vergleich zwischen benachbarten Volumenabtastpunkten bei örtlich und zeitlich fixiertem und damit statischem Zielvolumen und örtlich und zeitlich bewegtem und damit dynamischem Zielvolumen.

Fig. 4 zeigt eine Prinzipskizze einer Ausführungsform der Erfindung unter Bestrahlung eines Tumorgewebes im Kopfbereich eines Patienten.

Fig. 5 zeigt eine Prinzipskizze einer Ionenbremseinrichtung mittels variablem Wasservolumens.

Fig. 6 zeigt eine Prinzipskizze einer weiteren Ausführungsform der Erfindung unter Bestrahlung des Tumorgewebes im Kopfbereich eines Patienten.

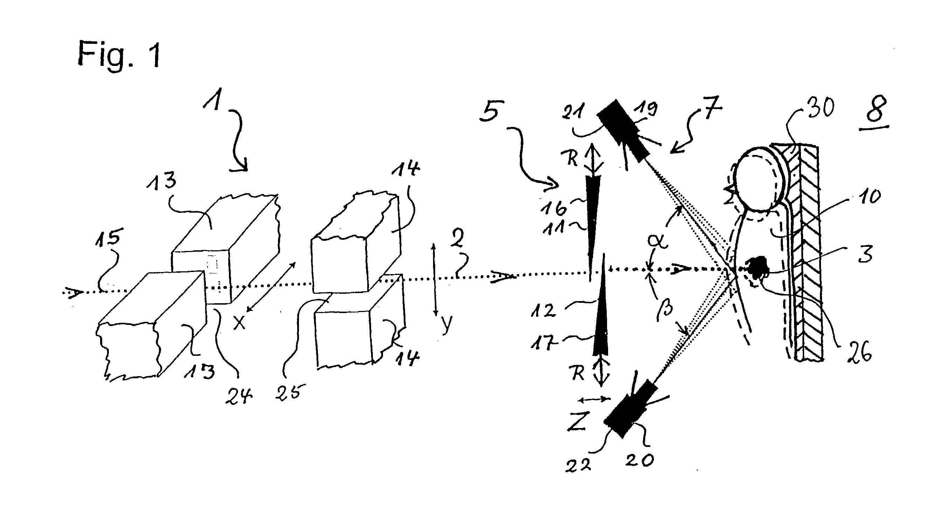

Fig. 1 zeigt eine Prinzipskizze einer Ausführungsform der Erfindung unter Bestrahlung eines Tumorgewebes 3 im Thoraxbereich 23 eines Patienten 10. Dazu weist die Vorrichtung einen Ionenstrahl 2 auf, der aus seiner Ionenstrahlachse 15 durch eine Ionenstrahl-Ablenkeinrichtung 1 orthogonal zur Ionenstrahlachse 15 abgelenkt wird, und zwar in einer X-Richtung beim Durchlaufen eines Spaltes 24 eines Elektromagneten 13 und in Y-Richtung beim Durchlaufen eines Spaltes 25 eines Elektromagneten 14, wobei die Spalte senkrecht aufeinanderstehen.

Der Ionenstrahl durchläuft ferner vor dem Auftreffen auf das Tumorgewebe 3 eines Patienten eine elektromagnetisch in Pfeilrichtung R angetriebene Ionen-Abbremseinrichtung 11, 12, die als eine Tiefenabtast-Anpassungsvorrichtung 5 zur Anpassung der Reichweite des Ionenstrahls 2 eingesetzt ist und eine schnellere Tiefenanpassung als eine nichtgezeigte Energiesteuereinrichtung aufweist, mit der die Energie des Ionenstrahls vor dem Eintreten in die Spalte 24, 25 der Elektromagnete 13 und 14 gesteuert wird.

Die nicht gezeigte Ionenstrahl-Energiesteuereinrichtung bewirkt eine gestaffelte Tiefenabtastung des Tumorgewebes 3, wobei durch stufenweises Erhöhen der Energie der Ionenstrahl jeweils nach einer scheibenweisen Flächenabtastung tiefer in das Tumorgewebe eindringt, so daß letztendlich das gesamte Tumorgewebe durch scheibenweise Tiefenabtastung des Ionenstrahls zerstört wird.

Bei Bewegung des hier skizzierten Patienten 10 in eine Lage, die mit gestrichelten Linie gezeichnet ist, verschiebt sich auch die Lage des Tumorgewebes 3, so daß bei einer statischen Bestrahlung, die nicht der Patientenbewegung folgen kann, gesundes Gewebe bestrahlt und zerstört würde.

Um dieses zu vermeiden, weist die Vorrichtung in Fig. 1 eine Bewegungserfassungseinrichtung 7 zur Erfassung einer zeitlichen und örtlichen Änderung der Lage des Tumorgewebes 3 in einem Behandlungsraum 8 auf. Diese Bewegungserfassungseinrichtung 7, die in dieser Ausführungsform der Erfindung aus zwei Präzisionsvideokameras 21 und 22 besteht, verfolgt die Bewegung von Markierungen auf einem Körperbereich des Patienten 10 und steht mit einer Bildauswerteeinrichtung in Verbindung, welche die erfaßten Änderungswerte der Markierungen mit der zeitlichen und örtlichen Änderung der Lage des Tumorgewebes 3 korreliert.

Eine in Fig. 1 nichtgezeigte Steuereinrichtung steuert sowohl die Ablenkeinrichtung 1 mit den beiden Elektromagneten 13 und 14 als auch die Tiefenabtast-Anpassungsvorrichtung mit der Ionen-Abbremseinrichtung 11, 12 zur Nachführung einerseits der Ionenstrahlrichtung und andererseits der Ionenstrahlreichweite beim Abtasten des Tumorgewebes unter zeitlicher und örtlicher Lageveränderung des Tumorgewebes 3 im Behandlungsraum 8. Mit der in Fig. 1 gezeigten Vorrichtung wird eine höhere Präzision der Strahlapplikation und damit ein verbesserter klinischer Erfolg in der mehr als hundertjährigen Geschichte der Entwicklung der Strahlentherapie erzielt.

Die fortgesetzte Präzisionssteigerung hat zur Verwendung dieses Scanningsystems aus zwei senkrecht zueinander angeordneten Elektromagnete geführt, durch deren Magnetspalte ein Ionenstrahl geführt und abgelenkt wird. Bei der Anwendung der Vorrichtung nach Fig. 1 wird das Zielvolumen, nämlich das Tumorgewebe 3, mit einem feinen Strahl von Ionen unter variabler Intensität abgerastert. Der Durchmesser dieses Ionenstrahls liegt im Millimeterbereich und die Präzision, mit der das Zielvolumen mit einer Ionendosis belegt werden kann, liegt ebenfalls im Bereich von wenigen Millimetern.

Bei einer Verschiebung des Zielvolumens 26 während der Bestrahlung kommt es zu einer Abweichung zwischen dem aktuellen Strahlenschwerpunkt und dem aktuellen Zielpunkt und damit zu einer Fehlbestrahlung im Inneren des Zielvolumens 26. Damit ist eine lokale Unterdosierung oder Überdosierung verbunden. Deshalb können Scanningverfahren ohne die erfindungsgemäße Vorrichtung nach Fig. 1 gegenwärtig bei bewegten Zielvolumina 26 nicht angewandt werden.

Andere Bestrahlungsvorrichtungen arbeiten mit stark aufgeweitetem Strahlenbündel und mit einem ebenfalls sehr stark verbreitetem Dosismaximum in der Tiefe. Derartig aufgeweitete Strahlenbündel können das ganze Zielvolumen ohne flächige Abtastung scheibenweise erfassen und aufgrund des großen Bestrahlungsfeldes keine Dosisinhomogenitäten im Inneren des Zielvolumens erzeugen, wie es bei der Verwendung von scheibenweisen Abtastvorrichtungen der Fall, wenn das Zielvolumen bewegt wäre. Die Bewegung von Organen wirkt sich bei Vorrichtungen mit aufgeweitetem Strahlenbündel nur am Rand aus und kann daher durch eine Vergrößerung des Bestrahlungsvolumens kompensiert werden, so daß bewegte Teile des Ziels nicht mehr das Bestrahlungsvolumen verlassen. Dieses bedeutet jedoch umgekehrt, daß ein großer Bereich von gesundem Normalgewebe am Rande des Zielvolumens mitbestrahlt werden muß, so daß mit einem aufgeweiteten Strahlenbündel unter gleichzeitiger Bewegung des bestrahlten Körpers eine verminderte Präzision und gleichzeitig erhöhte negative Nebeneffekte für die Patienten die Folge sind.

Mit einer Vorrichtung, wie sie in Fig. 1 gezeigt wird, jedoch ohne eine Tiefenabtast-Anpassungsvorrichtung 5, können Organbewegungen nur dadurch berücksichtigt werden, wenn der Querschnitt des Ionenstrahls 2 deutlich vergrößert wird. Eine derartige Lösung bedeutet jedoch ebenfalls eine Verringerung der Präzision im lateralen Bereich, und im longitudinalen Dosisprofil findet keine Korrektur statt, da mit einer Strahlaufweitung die Bewegung in Strahlrichtung nicht korrigiert werden kann. Somit ergibt sich bei einer Strahlaufweitung zur Abdeckung der Organbewegungen noch immer eine inhomogene Dosisverteilung des inneren Zielvolumens in Strahlrichtung.

Eine weitere Möglichkeit, die Vorrichtung der Fig. 1 ohne eine Tiefenabtast-Anpassungsvorrichtung 5 anzuwenden und trotzdem Organbewegungen zu berücksichtigen, kann darin bestehen, daß mit Hilfe der Bewegungserfassungseinrichtung 7 periodische Bewegungen beispielsweise des Thoraxbereiches eines Patienten erfaßt werden und nur dann eine Bestrahlung erfolgt, wenn der Thorax identische Positionen einnimmt. Eine derartige Vorrichtung, bei der keine Tiefenanpassungsvorrichtung vorgesehen ist, jedoch die periodische Bewegung eines Thorax eines Patienten erfaßt wird, würde die Bestrahlungs- bzw. Behandlungszeit eines Patienten um ein Vielfaches verlängern, da für jeden Volumenpunkt bei der scheibenweise Abtastung des Tumorvolumens zunächst die identische Position des Tumors abzuwarten ist. Lediglich eine Strahlaufweitung kann hier die Behandlungszeit auf realistische Größen vermindern, was wiederum, wie oben erwähnt, mit einem Verlust von Präzision verbunden ist.

Somit erweist sich die erfindungsgemäße Vorrichtung als das Konzept, mit dem optimal die Eindringtiefe des Ionenstrahls den Organbewegungen eines Patienten angepaßt werden kann, so daß mit hoher Präzision ein Tumorgewebe bei zeitlicher und örtlicher Lageänderung millimetergenau bestrahlt werden kann.

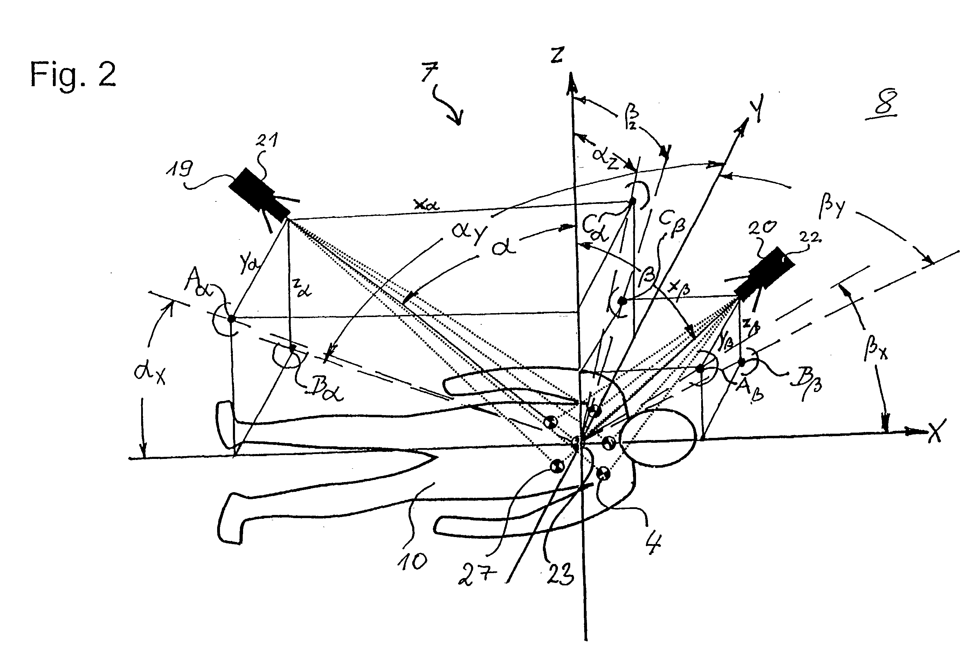

Fig. 2 zeigt eine Prinzipskizze einer Ausführungsform einer Bewegungserfassungseinrichtung 7. Diese Ausführungsform erfaßt die Bewegung eines Körperbereichs eines Patienten 10 mittels zweiter Präzisionsvideokameras 21 und 22, die Markierungen 4 auf dem Thorax eines Patienten 10 aus zwei unterschiedlichen Raumwinkeln $\alpha$ und $\beta$ erfassen und einer nichtgezeigten Bildauswerteeinheit zuführen. Die Markierungen 4 sind so gewählt, daß sie einerseits in der Nähe des zu bestrahlenden Tumorgewebes angeordnet sind und andererseits die Thoraxbewegungen derart genau erfassen, daß über die zeitlichen und örtlichen Änderungen der Markierungen 4 auf die zeitlichen und örtlichen Lageverschiebungen des Tumorgewebes geschlossen werden kann.

Die Raumwinkel $\alpha$ und $\beta$ weisen in dem karthesischen Koordinatensystem des Bestrahlungsraums 8 mit den Koordinatenrichtungen X, Y und Z Raumwinkelkomponenten $\alpha_x$, $\alpha_y$ und $\alpha_z$ für den Raumwinkel $\alpha$ und $\beta_x$, $\beta_y$ und $\beta_z$ für den Raumwinkel $\beta$ auf. Mit diesen Komponenten sind die Raumwinkel $\alpha$ und $\beta$ eindeutig im Bestrahlungsraum 8 mit dem Koordinatensystem X, Y und Z korreliert.

Die Position einer ersten Präzisionsvideokamera 21 weist dabei die Projektionspunkte $A_\alpha$, $B_\alpha$ und $C_\alpha$ auf, wobei der Projektionspunkt $A_\alpha$ die von den Koordinaten X und Z aufgespannte Ebene, der Projektionspunkt $B_\alpha$ die von den Koordinaten Y und X aufgespannte Ebene und der Projektionspunkt $C_\alpha$ die von den Koordinaten Z und Y aufgespannte Ebene durchstößt. Die Position der zweiten Kamera 22 weist die Projektionspunkt $A_\beta$, $B_\beta$ und $C_\beta$ auf, wobei der Projektionspunkt $A_\beta$ die Ebene, die von den Koordinaten X und Z aufgespannt wird, der Projektionspunkt $B_\beta$ die Ebene, die von den Koordinaten Y und X aufgespannt wird, und der Projektionspunkt $C_\beta$ die Ebene, die von den Koordinaten Z und Y aufgespannt wird, durchstößt. Über diese Projektionspunkte ist ebenfalls die Position der Präzisionskameras 21 und 22 im Bestrahlungsraum 8 eindeutig definiert, wobei die Koordinaten des Raumwinkels $\alpha$ der ersten Präzisionsvideokamer 21 $x_\alpha$, $y_\alpha$ und $z_\alpha$ betragen und die Koordinaten des Raumwinkels $\beta$ bei der Position der zweiten Präzisionsvideokamera 1 $x_\beta$, $y_\beta$ und $z_\beta$ sind.

Die größten Organbewegungen und damit die größten zeitlichen und örtlichen Änderungen der Markierung 4 ergeben sich bei einem Patienten im Lungenbereich während der Atmung. Im mittleren Thoraxbereich 23 werden Verschiebungen mit Amplituden von bis zu 1 cm und am Rande der Lunge von bis zu 3 cm festgestellt. Diese Verschiebungen durch die Atmung sind periodisch. Die Verschiebung der inneren Strukturen ist mit den Bewegungen der Körperoberfläche korreliert. Deshalb liefert eine optische Überwachung und Erfassung der Körperoberfläche mittels der Präzisionsvideokameras 21 und 22 jeweils aktuelle Ortskoordinaten der inneren Strukturen. Dazu können als Markierungen 4 Farbstriche, Farbpunkte oder Leuchtelemente wie Leuchtdioden auf der Körperoberfläche angebracht werden. Somit kann vorteilhafterweise ohne invasiven Eingriff die Geometrie im Patienteninneren zu jedem Zeitpunkt erfaßt werden und damit auch ein zeitlicher Verlauf der Verschiebungen der inneren Strukturen sowie des Tumorgewebes und die Geschwindigkeit der jeweiligen Bewegungen erfaßt werden.

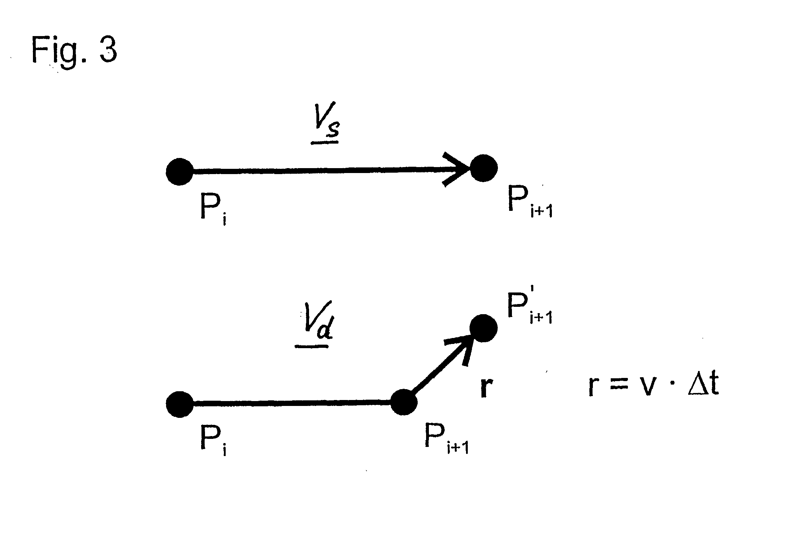

Fig. 3 zeigt einen Vergleich zwischen benachbarten Volumenabtastpunkten $P_i$ und $P_{i+1}$ bzw. $P'_{i+1}$ bei örtlich und zeitlich fixiertem und damit statischem Zielvolumen $V_s$ bzw. örtlich und zeitlich bewegtem und damit dynamischem Zielvolumen $V_d$. Die Erfassung der Bewegung der inneren Strukturen in Korrelation zur Oberfläche muß vor der Bestrahlung bekannt sein. Dieses kann aus Modellrechnungen oder auch aus Messungen hervorgehen.

Wird von einer Momentanaufnahme zum Zeitpunkt t = 0 ausgegangen, so kann bei einer Kurzzeitaufnahme ähnlich wie beim Rasterscanverfahren für ein nichtbewegtes Objekt das Zielvolumen in Schichten mit einer Tiefenkoordinate $z_i$ gleicher Teilchenreichweite zerlegt und jede Schicht mit einem lateralen Netz in X- und Y-Richtung mit Referenzbildpunkten, die Volumenabtastpunkten $P_i$ ($x_i$, $y_i$, $z_i$) überziehen. Während der Bestrahlung werden diese Bildpunkte aufgrund der Bewegung in eine Position $P'_i$ ($x_i + \Delta x_i(t)$ $y_i + \Delta y_i(t)$ $z_i + \Delta z_i(t)$) verschoben. Die Abweichungen oder Verschiebungen $\Delta x$, $\Delta y$ und $\Delta z$ ergeben sich aus der dreidimensionalen Geschwindigkeitsverteilung der Organbewegung in der Zeit $\Delta t$, die zur Dosisapplikation eines Volumenpunktes P des Tumorgewebes 3 nötig ist. Bei einem maximalen Hub des Thoraxbereichs 23 von 3 cm und einer Atemfrequenz von etwa 0,5 Hz, das heißt einer Dauer von 2 s, ergibt sich für die Organbewegungen beispielsweise eine Geschwindigkeit von etwa $v_{Organ}$ = 3 cm/s.

Die Bildpunkte $P_i$, die nun zu Volumenabtastpunkten werden, liegen bei einem lateralen und longitudinalen Scanverfahren 1 bis 3 mm auseinander, das heißt, nach einer Bestrahlungsdosis im Punkt $P_i$ wird der nächste benachbarte Punkt in 1 bis 3 mm Entfernung $P_{i+1}$ angefahren und in diesem Volumenabtastpunkt erneut eine Strahlendosis eingebracht. Die Zeit zur Applikation der Dosis in einem Volumenabtastpunkt $P_i$ oder $P_{i+1}$ beträgt unter 10 ms. Folglich bewegt sich der Zielpunkt in diesen 10 ms maximal um 0,3 mm, also sehr viel weniger als der Abstand zwischen zwei Volumenabtastpunkten $P_i$ und $P_{i+1}$. Da sich der aktuell bestrahlte Volumenabtastpunkt $P_i$ während der Bestrahlung um weniger als die Unschärfe der Bestrahlung bewegt, ist es nicht nötig, den Punkt $P_i$ während der jeweiligen Bestrahlung zu verschieben. Nach der Bestrahlung von $P_i$ muß der Strahl auf einen Punkt $P'_{i+1}$ wandern, der vom ursprünglich geplanten Punkt $P_{i+1}$ entsprechend der Koordinatenbewegung des Organs entfernt ist. Diese Organbewegung wird in Fig. 3 mit r bezeichnet und ergibt sich aus der Geschwindigkeit $r = v_{Organ} \cdot \Delta t$. Die tatsächliche Position des (i+1)-ten Punktes ist:

$$P'_{i+1} = (x_{i+1} + \Delta x_{i+1} (t), y_{i+1} + \Delta y_{i+1} (t), z_{i+1} + \Delta z_{i+1} (t)).$$

Die Abweichung oder Verschiebung der sich bewegenden Punkte $P'i$ von dem ursprünglich statischen Netz der Volumenabtastpunkte $P_i$ ergibt sich aus der Verschiebung während der Bestrahlungszeit. Bei den üblichen zyklischen und periodischen Bewegungen wie Atmung, Herzfrequenz usw. durchlaufen diese Punkte ebenfalls eine zyklische Kurve, die mit der Bewegung der Körperoberfläche korreliert werden kann. Deshalb ist eine Parametrierung des Weges mit der Zeit möglich. Nichtzyklische Vorgänge können mit der erfindungsgemäßen Vorrichtung und dem erfindungsgemäßen Verfahren zur Bestrahlung eines Tumorgewebes eines Patienten mittels Ionenstrahl nur geregelt werden, wenn der Bewegungsablauf nicht spontan auftritt, sondern mit einer Geschwindigkeit, die wesentlich langsamer ist als die Abtastgeschwindigkeit des Ionenstrahls. Bei plötzlichen hastigen Bewegungen, wie sie beispielsweise bei einem Hustenanfall auftreten, muß die Vorrichtung kurzfristig abschaltbar sein, um gesundes Gewebe vor einer Fehldosierung zu schützen.

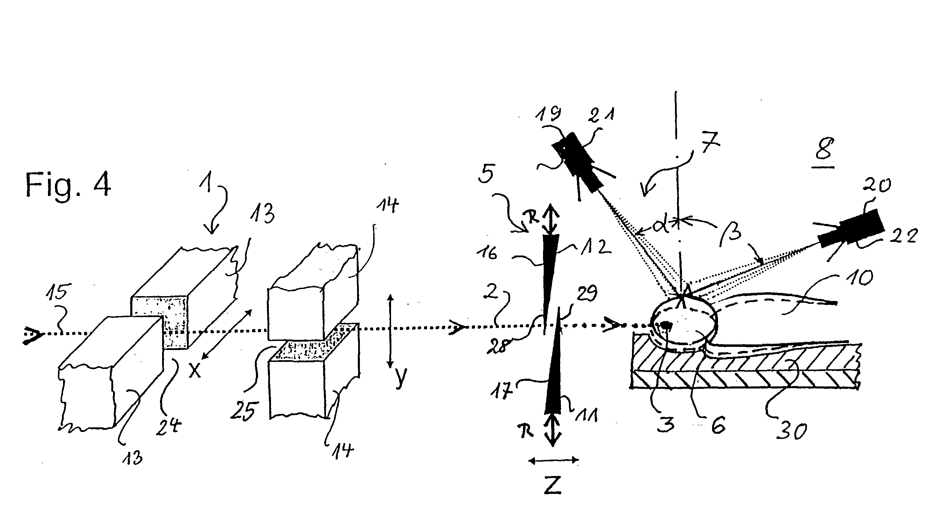

Fig. 4 zeigt eine Prinzipskizze einer Ausführungsform der Erfindung unter Bestrahlung eines Tumorgewebes 3 im Kopfbereich 6 eines Patienten 10. Die Vorrichtung der Fig. 4 entspricht im wesentlichen der Vorrichtung nach Fig. 1 und weist ebenso zwei Elektromagnete 13 und 14 zur Ablenkung des Ionenstrahls 2 aus seiner Achsrichtung 15 auf, wobei der Ionenstrahl in seiner Reichweite durch im Profil keilförmige Ionen-Abbremsplatten 16 und 17 gesteuert wird. Die überlappenden Bereiche 28 und 29 decken mindestens den gesamten Bestrahlungsbereich ab, wobei durch Aufeinanderzufahren der keilförmigen Profile der Abbremsplatten 16 und 17 der Abbremsweg des Ionenstrahls 2 durch die Abbremsplatten 16 und 17 vergrößert und damit die Reichweite des Ionenstrahls vermindert wird. Durch Auseinanderfahren der keilförmigen Abbremsplatten wird der Abbremsweg vermindert und damit die Reichweite des Ionenstrahls vergrößert.

Die Bewegungsrichtung der keilförmigen Abbremsplatten 16 und 17 wird durch die Pfeile R in Fig. 4 angedeutet. Die verschieblichen Abbremsplatten mit keilförmigem Profil werden in dieser Ausführungsform mit einem leistungsstarken Linearmotor angetrieben, so daß eine strahlintensive kontrollierte Tiefenanpassung vollzogen werden kann. Für den Linearantrieb weist die Tiefenabtast-Anpassungsvorrichtung 5 ein elektronisches Steuersystem auf, das mit der Bewegungserfassungseinrichtung 7 und der Ablenkeinrichtung 1 zusammenwirkt. Um eine schnelle Reaktion zu gewährleisten, sind die Läufer des Linearmotors, der auf einem Schlitten die Abbremsplatten 16 und 17 trägt, luftgelagert, und die Motorströme des Linearmotors werden über eine Schrittmotorsteuerung gesteuert.

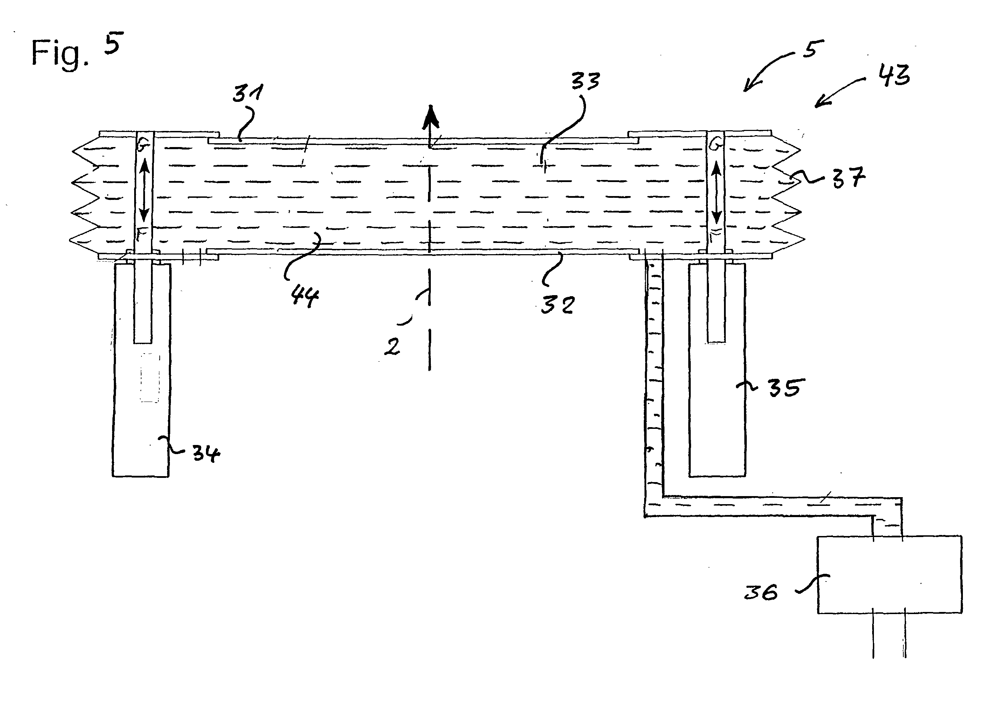

Fig. 5 zeigt eine Prinzipskizze einer Ionen-Abbremseinrichtung 43 mittels variablem Wasservolumen. Komponenten mit gleichen Funktionen wie in den vorhergehenden Figuren werden mit gleichen Bezugszeichen gekennzeichnet und nicht extra erläutert.

Das Bezugszeichen 44 kennzeichnet eine Wasserschicht, die zwischen zwei transparenten Platten 31 und 32 eingeschlossen ist. Von den transparenten Platten 31, 32 ist die Platte 31 mittels Linearmotoren 34 und 35 bewegbar. Die Zahl der Linearmotoren kann beliebig erhöht werden, um die Geschwindigkeit der Verschiebung der Platte 31 zu erhöhen. Die Wasserschicht 44 ist seitlich am Auslaufen durch einen Faltenbalg 37 gesichert. Um das Wasservolumen aufzufangen bzw. Wasser zu ergänzen je nach Bewegungsrichtung in Pfeilrichtungen G und F ist ein Ausgleichsbehälter 36 vorgesehen, der hydraulisch die Linearmotoren 34 und 35 dadurch unterstützt, daß beim Vergrößern der Wasserschicht 44 Wasser hinzugepumpt wird und beim Verkleinern der Dicke der Wasserschicht 44 Wasser abgesaugt wird. Das Bezugszeichen 33 kennzeichnet den mit Wasser gefüllten Zwischenraum. Der Ionenstrahl 2 wird zum Abbremsen durch die Wasserschicht 44 geschickt und muß dabei auch die transparenten Platten 31 und 32, die aus Glas oder Plexiglas bestehen können, durchdringen. Die geringste Abbremsung wird erreicht, wenn die beiden Platten 31 und 32 aufeinander liegen. Dann weisen sie auch eine äußerst geringe Dicke auf, die das Streuen des Ionenstrahls minimiert.

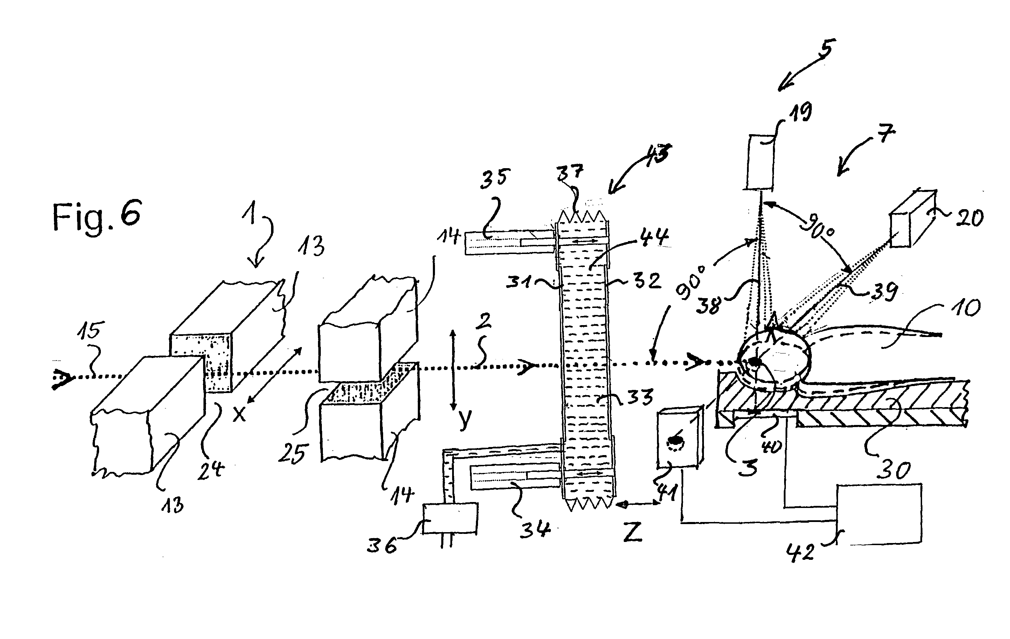

Fig. 6 zeigt eine Prinzipskizze einer weiteren Ausführungsform der Erfindung unter Bestrahlung eines Tumorgewebes 3 im Kopfbereich eines Patienten. Komponenten mit gleichen Funktionen wie in den vorhergehenden Figuren werden mit gleichen Bezugszeichen gekennzeichnet und nicht extra erörtert.

Diese weitere Ausführungsform unterscheidet sich von der Ausführungsform nach Fig. 4 dadurch, daß als Ionen-Abbremseinrichtung 43 nicht das Keilsystem eingesetzt wird, sondern die in Fig. 5 gezeigte Abbremseinrichtung 43. Ferner unterscheidet sich die weiter Ausführungsform in Fig. 6 von den Ausführungsformen in Fig. 1 und Fig. 4 dadurch, daß als Bewegungserfassungssystem ein System aus mindestens zwei Röntgenröhren eingesetzt wird. Die Meßaufnehmer 19 und 20 sind Röntgenröhren, die Röntgenstrahlen 38 und 39 in einem rechten Winkel zueinander auf das Tumorgewebe 3 des Patienten 10 richten. Diese Röntgenstrahlen werden von Röntgenblitzen mit niedriger Leistung in einer Frequenz von beispielsweise 20 Hz auf das Tumorgewebe gerichtet und von entsprechenden Bildverstärkerplatten oder Sensorplatten 40 und 41 empfangen, die ihre Signale an die Auswerteeinheit 42 weitergeben. Dieses Bewegungserfassungssystem mit Röntgenstrahlen kann unmittelbar im Körperinneren die Bewegungen des Tumorvolumens verfolgen und somit sehr präzise die Abbremseinrichtung 43 steuern.

1. Durchführungsbeispiel des Verfahrens zur Anwendung der erfindungsgemäßen Vorrichtung

Bei einem ersten Durchführungsbeispiel des Verfahrens wird vor der Bestrahlung wird die Oberfläche des Patienten mit signifikanten Markierungen versehen, wie z.B. Farbmarkierungen auf der Haut oder Leuchtdioden usw.. Der Patient wird beispielsweise auf einem Schaumstoffbett, wie es in Fig. 1 und Fig. 4 mit der Bezugsnummer 30 zu sehen ist, gelagert, das seinem Körper angepaßt ist. Damit kann ohne Zwang eine Lagergenauigkeit von ca. 1 cm erreicht werden. In Bestrahlungsposition, wie es die Figuren Fig. 1 und Fig. 4 zeigen, wird der Patient 10 mit einem Präzisionsvideosystem von mindestens zwei Präzisionsvideokameras 21 und 22 aus verschiedenen Raumrichtungen unter Raumwinkeln $\alpha$ und $\beta$ überwacht, welche die Position der Markierungen 4 (vergleiche Fig. 2) als Funktion der Zeit aufnehmen und eine zeitabhängige Korrekturfunktion der Bildpunkte bzw. Volumenabtastpunkte $P_i'(t)$ erstellen.

Um eine schnelle Verschiebung des Ionenstrahls 2 in drei Dimensionen zu erreichen, wird der laterale intensitätsgesteuerte Rasterscanner aus zwei Elektromagneten 13 und 14 mit einer Tiefenabtast-Anpassungsvorrichtung 5 kombiniert, da eine schnelle Energievariation durch eine Ionenstrahl-Energiesteuerein-richtung während der Bestrahlung eines Volumenpunktes P von keinem Ionenbeschleuniger her möglich ist. Der laterale Scanteil, der wie oben erwähnt aus zwei Elektromagneten besteht, deren Ablenkrichtung senkrecht aufeinander und zur Strahlachse 15 angeordnet ist, wird durch schnelle Netzgeräte gesteuert, so daß eine schnelle seitliche Abtastanpassung in X- und Y-Richtung gewährleistet ist.

Zusätzlich zu der Abtasteinrichtung 1 in X- und Y-Richtung wird direkt vor dem Patienten eine elektromechanisch betriebene Tiefenabtast-Anpassungsvorrichtung angeordnet, die im wesentlichen aus zwei Keilen besteht, die gegenläufig auf einem Linearmotor montiert sind und das gesamte Bestrahlungsfeld überdecken. Diese Tiefenabtast-Anpassungsvorrichtung dient lediglich der Korrektur der Tiefen-Positionsveränderung der Bildpunkte durch Bewegung des Patienten oder der Patientenorgane. Diese Tiefenabtast-Anpassungsvorrichtung muß nicht die gesamte Tiefe des Zielvolumens ausleuchten.

Zur groben Tiefenvariation wird die Energievariation eines Synchrotrons oder eines anderen Beschleunigers herangezogen. Dabei wird der Strahlstrom mit einer im Strahlengang vor dem Patienten installierten Ionisationskammer überwacht und von einem Volumenabtastpunkt $P_i$ zum nächsten Abtastpunkt $P_{i+1}$ weitergeschaltet, wenn die nötige Teilchendosis pro Volumenabtastpunkt P erreicht ist. Gleichzeitig wird entsprechend der Präzisionsvideokameraüberwachung des Patienten die Oberflächenbewegung gemessen und daraus die Bewegung der inneren Strukturen im Zielvolumen berechnet.

Aufgrund der Wanderung der Zielpunkte in drei Dimensionen werden die Magnetwerte der Ablenkmagnete sowie die Tiefenanpassungswerte der keilförmigen Abbremsplatten korrigiert. Da der Strahl bei dieser Art des Verfahrens und der Vorrichtung während der Bestrahlung nicht unterbrochen wird, ergeben sich auch keine unbestrahlten Stellen im Tumorgewebe. Die Präzision im Inneren und auch der scharfe Randabfall einer statischen Bestrahlung wird somit auch für bewegte Organe unter dynamischer Bestrahlung erreicht.

Zusätzlich ist die Vorrichtung und das Verfahren auch gegenüber der Kompression von Voluminadosen invariabel. Bei einer Volumenkompression, z.B. in der Lunge, kommen die Bildpunkte dichter aneinander zu liegen. Dadurch erhöht sich die lokale Teilchenfluenz. Gleichzeitig wird durch die Kompression auch die Massendichte erhöht. Da die Dosis als Energiedeposition pro Dichte definiert ist, bleibt sie bei einer Kompression in erster Näherung erhalten. Das bedeutet, daß die Teilchenbewegung der einzelnen Strahlenpositionen während der Bestrahlung bei der erfindungsgemäßen Vorrichtung und dem Verfahren nicht korrigiert werden muß.

2. Durchführungsbeispiel des Verfahrens zur Anwendung der erfindungsgemäßen Vorrichtung

Bei einem zweiten Durchführungsbeispiel des Verfahrens werden statt der Markierung auf dem Körper des Patienten die zeitlichen und örtlichen Änderungen des Tumorgewebes unmittelbar durch Röntgenstrahlen erfaßt. Dazu werden nach der Ausrichtung des Patienten zwei Röntgenstrahlen auf das Tumorgewebe gerichtet, die senkrecht zum Ionenstrahl 2 angeordnet sind und die kurze Röntgenblitze bei niedriger Leistung liefern, um die Dosisbelastung des Patienten gering zu halten. Diese Blitze können mit einer Frequenz von etwa 20 Hz auf das Tumorgewebe gerichtet sein. Die Röntgenstrahlrichtungen sind zueinander um 90° versetzt und gemeinsam sind sie orthogonal zum Ionenstrahl 2 angeordnet. Mit Hilfe der Röntgenstrahlblitze werden Bildverstärkerplatten belichtet, die ihre Signale an eine Auswerteeinheit 42 geben, wobei die Auswerteeinheit 42 die Abbremseinrichtung 43 steuern. Die Abbremseinrichtung 43 ist in diesem zweiten Durchführungsbeispiel der Erfindung eine Wasserschicht 33, deren Dicke variiert wird und die zwischen zwei transparenten Platten 31 und 32 angeordnet ist. Die schnelle Variation der Wasserschichtdicke wird durch Ändern des Zwischenraums 33 zwischen den beiden transparenten Platten 31 und 32 bewirkt. Diese Verschiebung wird von Linearmotoren 34 und 35 vorgenommen, wobei gleichzeitig ein Ausgleichsgefäß 36 für den Druckausgleich und den Volumenausgleich des Wassers sorgt. Alle anderen Bestrahlungsschritte zur Behandlung des Tumorvolumens entsprechen den Verfahrensschritten, die bereits im ersten Durchführungsbeispiel aufgeführt wurden.

**Bezugszeichenliste**

| | |
|---|---|
| 1 | Ionenstrahl-Ablenkeinrichtung |
| 2 | Ionenstrahl |
| 3 | Tumorgewebe |
| 4 | Markierung |
| 5 | Tiefenabtast-Anpassungsvorrichtung |
| 6 | Kopfbereich |
| 7 | Bewegungserfassungseinrichtung |
| 8 | Behandlungsraum |
| 9 | Ionenstrahlen-Energiesteuereinrichtung |
| 10 | Patient |
| 11, 12 | Ionen-Abbremseinrichtung |
| 13 | Elektromagnet für X-Ablenkung |
| 14 | Elektromagnet für Y-Ablenkung |
| 15 | Ionenstrahlachse |
| 16, 17 | Ionen-Abbremsplatten |
| 19, 20 | zwei Meßaufnehmer |
| 21, 22 | zwei Präzisionsvideokameras |
| 23 | Thoraxbereich |
| 24 | Spalt des Elektromagneten 13 |
| 25 | Spalt des Elektromagneten 14 |
| 26 | Zielvolumen |
| 27 | Rand der Lunge |
| 28, 29 | Überlappende Bereiche |
| 30 | Schaumstoffbett |
| 31, 32 | transparente Platten |
| 33 | Zwischenraum |
| 34, 35 | Linearmotoren |
| 36 | Ausgleichsbehälter |
| 37 | Faltenbalg |
| 38, 39 | Röntgenstrahlen |
| 40, 41 | Sensorplatten |
| 42 | Auswerteeinheit |
| 43 | Ionen-Abbremseinrichtung |
| 44 | Wasserschicht |

| | |
|---|---|
| $V_s$ | statisches Zielvolumen |
| $V_d$ | dynamisches Zielvolumen |
| $P, P_i$ | Volumenabtastpunkt |
| $P_{i+1}$ | zu $P_i$ benachbarter Volumenabtastpunkt |
| $\alpha, \beta$ | Raumwinkel |
| $\alpha_x, \alpha_y, \alpha_z$ | Komponenten des Raumwinkels $\alpha$ |
| $\beta_x, \beta_y, \beta_z$ | Komponenten des Raumwinkels $\beta$ |

$x_\alpha, y_\alpha, z_\alpha$      Koordinaten des Raumwinkels $\alpha$ bei der ersten Kameraposition

$x_\beta, y_\beta, z_\beta$      Koordinaten des Raumwinkels $\beta$ bei der zweiten Kameraposition

$A_\alpha, B_\alpha, C_\alpha$      Projektionspunkte auf die Ebenen $XZ(A_\alpha)$; $YX(B_\alpha)$; $ZY(C_\alpha)$ der ersten Kameraposition

$A_\beta, B_\beta, C_\beta$      Projektionspunkte auf die Ebenen $XZ(A_\beta)$; $YX(B_\beta)$ und $ZY(C_\beta)$ der zweiten Kameraposition

**Patentansprüche**

1. Vorrichtung zur Bestrahlung eines Tumorgewebes (3) eines Patienten (10) mittels Ionenstrahl (2), die

   - eine Ablenkeinrichtung (1) des Ionenstrahls (2) zum scheibenweise Flächenabtasten des Tumorgewebes (3) und
   - einen Beschleuniger mit einer Ionenstrahl-Energiesteuereinrichtung zur stufenweisen Tiefenabtastung des Tumorgewebes (3) aufweist,

   **dadurch gekennzeichnet, daß**
   die Vorrichtung weiterhin aufweist:

   - eine elektromechanisch angetriebene Ionen-Abbremseinrichtung (11, 12), die als eine Tiefenabtast-Anpassungsvorrichtung (5) zur Anpassung der Reichweite des Ionenstrahls (2) eingesetzt ist und eine schnellere Tiefenanpassung als die Energiesteuereinrichtung des Beschleunigers aufweist,
   - eine Bewegungserfassungseinrichtung (7) zur Erfassung einer zeitlichen und örtlichen Änderung der Lage des Tumorgewebes (3) in einem Behandlungsraum (8), und
   - eine Steuereinrichtung, welche die Ablenkeinrichtung (1) und die Tiefenabtast-Anpassungsvorrichtung (5) zur Nachführung der Ionenstrahlrichtung bzw. Ionenstrahlreichweite beim Abtasten des Tumorgewebes (3) unter zeitlicher und örtlicher Lageveränderung des Tumorgewebes (3) im Behandlungsraum (8) steuert.

2. Vorrichtung nach Anspruch 1, **dadurch gekennzeichnet, daß** die Ablenkvorrichtung (1) zwei Elektromagnete (13, 14) aufweist, die einen Ionenstrahl orthogonal zur Ionenstrahlachse (15) in einer X- und einer Y-Richtung, die ihrerseits senkrecht zueinander liegen, zum scheibenweisen Flächenabtasten des Tumorgewebes (3) ablenken.

3. Vorrichtung nach Anspruch 2, **dadurch gekennzeichnet, daß** die Elektromagnete durch reaktionsschnelle Netzgeräte gesteuert werden.

4. Vorrichtung nach einem der vorhergehenden Ansprüche, **dadurch gekennzeichnet, daß** die Vorrichtung Beschleuniger aufweist, mit denen die Energie des Ionenstrahls (2) einstellbar ist, so daß das Tumorgewebe (3) scheibenweise in der Tiefe gestaffelt bestrahlbar ist.

5. Vorrichtung nach einem der vorhergehenden Ansprüche, **dadurch gekennzeichnet, daß** die Tiefenabtast-Anpassungsvorrichtung (5) zur schnellen Tiefenabtastanpassung bei bewegtem Tumorgewebe (3) eine elektromechanisch betriebene Ionen-Abbremseinrichtung aufweist, die zwei im Querschnitt keilförmige Ionen-Abbremsplatten (16, 17) aufweist, die das gesamte Bestrahlungsfeld des Ionenstrahls (2) abdecken.

6. Vorrichtung nach Anspruch 5, **dadurch gekennzeichnet, daß** die Ionen-Abbremsplatten (16, 17) auf Linearmotoren montiert sind.

7. Vorrichtung nach Anspruch 5, **dadurch gekennzeichnet, daß** die Ionen-Abbremsplatten (16, 17) auf elektromagnetisch betätigbaren Schlitten angeordnet sind.

8. Vorrichtung nach einem der Ansprüche 5 bis 7, **dadurch gekennzeichnet, daß** die Ionen-Abbremsplatten mit ihrem keilförmigen Querschnitt überlappend im Bereich des Ionenstrahls (2) gegeneinander verschiebbar sind.

9. Vorrichtung nach einem der Ansprüche 1 bis 4, **dadurch gekennzeichnet, daß** die Tiefenabtast-Anpassungsvorrichtung (5) zur schnellen Tiefenabtastanpassung bei bewegtem Tumorgewebe (3) eine hydraulisch unterstützte Ionen-Abbremseinrichtung aufweist, bei der die Dicke einer Wasserschicht (30) zwischen zwei transparenten Platten (31, 32), durch welche der Ionenstrahl (2) geleitet wird, den Bewegungen des Tumorgewebes angepaßt ist.

10. Vorrichtung nach Anspruch 9, **dadurch gekennzeichnet, daß** die zwei transparenten Platten (31, 32) auf einander zu bewegbar sind und in ihrem Zwischenraum (33) Wasser aufweisen.

11. Vorrichtung nach Anspruch 9 oder 10, **dadurch gekennzeichnet, daß** der Abstand der transparenten Platten (31, 32) und somit die Dicke der Wasserschicht (33) mittels Linearmotoren (34, 35) einstellbar ist.

12. Vorrichtung nach einem der Ansprüche 9 bis 11, **dadurch gekennzeichnet, daß** die Tiefenabtast-Anpassungsvorrichtung (5) einen hydraulisch betriebenen Ausgleichsbehälter (36) für das Wasservolumen zwischen den transparenten Platten (31, 32) aufweist.

13. Vorrichtung nach einem der Ansprüche 9 bis 12, **dadurch gekennzeichnet, daß** ein Faltenbalg (37) zwischen den transparenten Platten (31, 32) angeordnet ist.

14. Vorrichtung nach einem der vorhergehenden Ansprüche, **dadurch gekennzeichnet, daß** die Bewegungserfassungseinrichtung (7) mindestens zwei Meßaufnehmer (19, 20) aufweist, die aus zwei Raumwinkeln ($\alpha$, $\beta$) in bezug auf eine Ionenstrahlachse (15), die zeitliche und örtliche Lage von Markierungen auf einem, ein Tumorgewebe (3) enthaltenden Körperbereich eines Patienten (10) erfassen.

15. Vorrichtung nach Anspruch 14, **dadurch gekennzeichnet, daß** die Meßaufnehmer (19, 20) Präzisionsvideokameras (21, 22) sind, die mit einer Bildauswerteeinheit zusammenwirken.

16. Vorrichtung nach einem der Ansprüche 1 bis 13, **dadurch gekennzeichnet, daß** die Bewegungserfassungseinrichtung (7) mindestens zwei Meßaufnehmer (19, 20) aufweist, die orthongonal zum Ionenstrahl und zueinander senkrecht angeordnet sind, wobei die zeitliche und örtliche Änderung der Lage des Tumorgewebes durch kurze Impulse von Röntgenstrahlen (38, 39) überwacht werden, und wobei zum Erfassen der Bilder des Tumorgewebes die Bewegungserfassungseinrichtung (7) entsprechend angeordnete Sensorplatten (40, 41) und eine Auswerteeinheit (42) aufweist.

17. Vorrichtung nach einem der vorhergehenden Ansprüche, **dadurch gekennzeichnet, daß** eine Ionisationskammer mit schneller Auslese zur Überwachung der Intensität des Ionenstrahlstroms als Transmissionszähler im Strahlengang des Ionenstrahls (2) angeordnet ist.

18. Vorrichtung nach Anspruch 17, **dadurch gekennzeichnet, daß** die Ionisationskammer zwischen der Ablenkeinrichtung (1) und der Tiefenabtast-Anpassungsvorrichtung (5) angeordnet ist.

## Claims

1. Apparatus for irradiating tumour tissue (3) of a patient (10) by means of an ion beam (2), having

   - a deflecting device (1) for the ion beam (2) for slice-wise and area-wise scanning of the tumour tissue (3) and
   - an accelerator having an ion beam energy control device for step-wise and depth-wise scanning of the tumour tissue (3),

   **characterised in that**
   the apparatus further has:

   - an electromechanically driven ion-braking device (11, 12), which is used as a depth-wise scanning adaptation apparatus (5) for adapting the range of the ion beam (2) and which has faster depth-wise adaptation than the energy control device of the accelerator,
   - a movement detection device (7) for detecting a temporal and positional change in the location of the tumour tissue (3) in a treatment space (8), and
   - a control device which controls the deflecting device (1) and the depth-wise scanning adaptation apparatus (5) for adjusting the ion beam direction and the ion beam range, respectively, when scanning the tumour tissue (3) in the course of temporal and positional change in the location of the tumour tissue (3) in the treatment space (8).

2. Apparatus according to claim 1, **characterised in that** the deflecting device (1) has two electromagnets (13, 14), which, for slice-wise and area-wise scanning of the tumour tissue (3), deflect an ion beam orthogonally to the ion beam axis (15) in an X direction and a Y direction, which are in turn located perpendicular to one another.

3. Apparatus according to claim 2, **characterised in that** the electromagnets are controlled by fast-reacting power units.

4. Apparatus according to one of the preceding claims, **characterised in that** the apparatus has accelerators by means of which the energy of the ion beam (2) is arranged to be so adjusted that the tumour tissue (3) can be irradiated slice-wise, staggered in terms of depth.

5. Apparatus according to one of the preceding claims, **characterised in that** the depth-wise scanning adaptation apparatus (5) has, for fast depth-wise scanning adaptation in the case of moving tumour tissue (3), an electromechanically operated ion-braking device which has two ion-braking plates (16, 17), which in cross-section are wedge-shaped and which cover the entire irradiation field of the ion beam (2).

6. Apparatus according to claim 5, **characterised in that** the ion-braking plates (16, 17) are mounted on linear motors.

7. Apparatus according to claim 5, **characterised in that** the ion-braking plates (16, 17) are arranged on electromagnetically actuatable carriages.

8. Apparatus according to one of claims 5 to 7, **characterised in that** the ion-braking plates are arranged to be displaced in opposite directions, their wedge-shaped cross-sections overlapping in the region of the ion beam (2).

9. Apparatus according to one of claims 1 to 4, **characterised in that** the depth-wise scanning adaptation apparatus (5) has, for fast depth-wise scanning adaptation in the case of moving tumour tissue (3), a hydraulically assisted ion-braking device wherein the thickness of a water layer (30) between two transparent plates (31, 32), through which the ion beam (2) is directed, is adapted to the movements of the tumour tissue.

10. Apparatus according to claim 9, **characterised in that** the two transparent plates (31, 32) are arranged to be moved towards one another and have water in their intermediate space (33).

11. Apparatus according to claim 9 or 10, **characterised in that** the spacing between the transparent plates (31, 32) and, consequently, the thickness of the water layer (33) are arranged to be adjusted by means of linear motors (34, 35).

12. Apparatus according to one of claims 9 to 11, **characterised in that** the depth-wise scanning adaptation apparatus (5) has a hydraulically operated compensation tank (36) for the water volume between the transparent plates (31, 32).

13. Apparatus according to one of claims 9 to 12, **characterised in that** a bellows (37) is arranged between the transparent plates (31, 32).

14. Apparatus according to one of the preceding claims, **characterised in that** the movement detection device (7) has at least two measurement sensors (19, 20), which detect, from two spatial angles ($\alpha$, $\beta$) in relation to an ion beam axis (15), the temporal and positional location of markings on a region of the body of a patient (10) that contains tumour tissue(3).

15. Apparatus according to claim 14, **characterised in that** the measurement sensors (19, 20) are precision video cameras (21, 22), which cooperate with an image-evaluating unit.

16. Apparatus according to one of claims 1 to 13, **characterised in that** the movement detection device (7) has at least two measurement sensors (19, 20), which are arranged orthogonally to ion beam and perpendicular to one another, the temporal and positional change in the location of the tumour tissue being monitored by short pulses of X-ray beams (38, 39), and the movement detection device (7) having, for detection of the images of the tumour tissue, correspondingly arranged sensor plates (40, 41) and an evaluating unit (42).

17. Apparatus according to one of the preceding claims, **characterised in that** an ionisation chamber having a fast read-out for monitoring the intensity of the ion beam flow is arranged as a transmission counter in the beam path of the ion beam (2).

**18.** Apparatus according to claim 17, **characterised in that** the ionisation chamber is arranged between the deflecting device (1) and the depth-wise scanning adaptation apparatus (5).

**Revendications**

**1.** Dispositif pour irradier un tissu tumoral (3) chez un patient (10) au moyen d'un faisceau d'ions (2), comportant

- un dispositif de déviation (1) du faisceau d'ions (2) pour le balayage plan par tranches du tissu tumoral (3) et
- un accélérateur avec un dispositif de contrôle de l'énergie du faisceau d'ions pour le balayage en profondeur par paliers du tissu tumoral (3),

**caractérisé par le fait que**
le dispositif présente en outre

- un dispositif de freinage d'ions (11, 12) entraîné par des moyens électro-magnétiques, qui est utilisé en tant que dispositif de réglage du balayage en profondeur (5) pour régler la portée du faisceau d'ions (2) et offre un réglage de profondeur plus rapide que le dispositif de contrôle d'énergie de l'accélérateur,
- un dispositif de détection de déplacement (7) pour détecter une modification dans le temps et dans l'espace de la position du tissu tumoral (3) dans une enceinte de traitement (8) et
- un dispositif de commande qui commande le dispositif de déviation (1) et le dispositif de réglage du balayage en profondeur (5) aux fins de corriger la direction du faisceau d'ions ou la portée du faisceau d'ions au cours du balayage du tissu tumoral (3) en fonction de la modification dans le temps et dans l'espace de la position du tissu tumoral (3) dans l'enceinte de traitement (8).

**2.** Dispositif selon la revendication 1, **caractérisé par le fait que** le dispositif de déviation (1) présente deux électro-aimants (13, 14) qui dévient un faisceau d'ions orthogonalement à l'axe (15) du faisceau d'ions dans des directions X et Y, elles-mêmes mutuellement perpendiculaires, à des fins de balayage plan par tranches du tissu tumoral (3).

**3.** Dispositif selon la revendication 2, **caractérisé par le fait que** les électro-aimants sont commandés par des appareils de réseau à réaction rapide.

**4.** Dispositif selon une des revendications précédentes, **caractérisé par le fait que** le dispositif comporte des accélérateurs, à l'aide desquels l'énergie du faisceau d'ions (2) peut être réglée de manière à irradier graduellement le tissu tumoral (3) par tranches successives en profondeur.

**5.** Dispositif selon une des revendications précédentes, **caractérisé par le fait que** le dispositif de réglage du balayage en profondeur (5), pour une adaptation rapide du balayage en profondeur en présence d'un déplacement du tissu tumoral (3), comporte un dispositif de freinage d'ions mû par des moyens électro-mécaniques, qui comporte deux plaques de freinage d'ions (16, 17) à section transversale cunéiforme, couvrant la totalité de la zone d'irradiation du faisceau d'ions.

**6.** Dispositif selon la revendication 5, **caractérisé par le fait que** les plaques de freinage d'ions (16, 17) sont montées sur des moteurs linéaires.

**7.** Dispositif selon la revendication 5, **caractérisé par le fait que** les plaques de freinage d'ions (16, 17) sont montées sur des chariots actionnables des moyens électro-magnétiques.

**8.** Dispositif selon une des revendications 5 à 7, **caractérisé par le fait que** les plaques de freinage d'ions à section transversale cunéiforme peuvent coulisser en direction l'une de l'autre en se chevauchant dans la région du faisceau d'ions (2).

**9.** Dispositif selon une des revendications 1 à 4, **caractérisé par le fait que** le dispositif de réglage du balayage en profondeur (5), pour une adaptation rapide du balayage en profondeur en présence d'un déplacement du tissu tumoral (3), comporte un dispositif de freinage d'ions à action renforcée par des moyens hydrauliques, dans lequel l'épaisseur d'une couche d'eau (30) entre deux plaques (31, 32) transparentes traversées par le faisceau d'ions, est adaptée aux déplacements du tissu tumoral.

**10.** Dispositif selon la revendication 9, **caractérisé par le fait que** les deux plaques (31, 32) transparentes peuvent être déplacées en direction l'une de l'autre et que de l'eau est contenue dans l'espace intermédiaire (33) entre les plaques.

**11.** Dispositif selon la revendication 9 ou 10, **caractérisé par le fait que** la distance entre les deux plaques (31, 32) transparentes et par conséquent l'épaisseur de la couche d'eau (33) peut être réglée à l'aide de moteurs linéaires (34, 35).

**12.** Dispositif selon une des revendications 9 à 11, **caractérisé par le fait que** le dispositif de réglage du balayage en profondeur (5) comporte un réservoir de compensation (36) hydraulique pour le volume d'eau entre les plaques (31, 32) transparentes.

**13.** Dispositif selon une des revendications 9 à 12, **caractérisé par le fait qu'**un soufflet (37) est disposé entre les plaques (31, 32) transparentes.

**14.** Dispositif selon une des revendications précédentes, **caractérisé par le fait que** le dispositif de détection de déplacement (7) comporte au moins deux détecteurs (19, 20) qui, sous deux angles solides ($\alpha$, $\beta$) par rapport à un axe de faisceau d'ions (15), mesurent la position dans le temps et dans l'espace de repères sur une zone du corps d'un patient (10) présentant un tissu tumoral (3).

**15.** Dispositif selon la revendication 14, **caractérisé par le fait que** les détecteurs (19, 20) sont des caméras vidéo de précision (21, 22) qui coopèrent avec une unité de traitement d'image.

**16.** Dispositif selon une des revendications 1 à 13, **caractérisé par le fait que** le dispositif de détection de déplacement (7) comporte au moins deux détecteurs (19, 20) qui sont disposés orthogonalement au faisceau d'ions et perpendiculairement entre eux, la modification dans le temps et dans l'espace de la position du tissu tumoral étant surveillée par des impulsions courtes de rayons X (38, 39) et le dispositif de détection de déplacement (7) comportant pour la saisie des images du tissu tumoral des plaques de détecteur (40, 41) disposées de manière appropriée et une unité de traitement (42).

**17.** Dispositif selon une des revendications précédentes, **caractérisé par le fait qu'**une chambre d'ionisation à accès rapide est disposée sur le trajet du faisceau d'ions (2) en tant que compteur de transmission pour la surveillance de l'intensité du faisceau d'ions.

**18.** Dispositif selon la revendication 17, **caractérisé par le fait que** la chambre d'ionisation est disposée entre le dispositif de déviation (1) et le dispositif de réglage de balayage en profondeur (5).

Fig. 1

Fig. 2

EP 1 294 445 B1

Fig. 3

$$\underline{V_s}$$

●————————————————▶ ●
$P_i$                          $P_{i+1}$

$$\underline{V_d}$$

$P'_{i+1}$

●————————————————● 
$P_i$                    $P_{i+1}$    **r**

$$r = v \cdot \Delta t$$

EP 1 294 445 B1

Fig. 4

EP 1 294 445 B1

Fig. 5

EP 1 294 445 B1

Fig. 6